# EUROPEAN PATENT SPECIFICATION

(11) **EP 1 362 123 B1**
(45) Date of publication and mention of the grant of the patent: **28.06.2006**
(21) Application number: 02712099.7
(22) Date of filing: 20.02.2002
(51) Int. Cl.: C12Q 1/68

(54) **SECURITY SYSTEM**
SICHERHEITSSYSTEM
SYSTEME DE SECURITE

(30) Priority: 20.02.2001 GB 0104163
(43) Date of publication of application: 19.11.2003
(73) Proprietor: Crime Solutions Limited, Coventry CV5 9EN (GB)
(72) Inventor: BROWN, Tom, Chilworth, Southampton SO16 7JD (GB); THELWELL, Nichola, Derbyshire SK13 7 QU (GB); MAXWELL, Paula, Coventry CV5 6GE (GB); MAXWELL, Paul, Coventry CV5 9EN (GB); WHITING, Paul, Coventry, West Midlands CV3 4HD (GB)
(74) Representative: MacLean, Martin Robert
(86) International application number: PCT/GB2002/000759
(87) International publication number: WO 2002/066678

(56) References cited:
- US-A- 5 763 176
- US-A- 5 942 391

## Description

The present invention relates to security markers for marking objects and people, methods of using the markers, methods of detecting the markers and security devices containing such markers. In particular it relates to an error-free system which will stand up in a Court of law and help the police and the Courts to convict criminals so creating a product that has excellent deterrent value. In particular, the security markers used comprise a nucleic acid molecule, specially designed in a security conscious manner to enable it to be used in this industry, that can be uniquely identified to a specific source of the security marker and so place, time and date.

Security markers for identifying the ownership of an article are known in the art. Some markers use a specific formulation of different chemical compounds to identify that an object is owned by somebody or was at a specific location. Such chemical markers are shown, for example, in GB 2245583. Such systems have only a limited number of identifying compounds, thus making it difficult to uniquely identify a large number of locations. Further, it is necessary to disperse a large amount of substance, so making them messy, impractical and open to possible cross-contamination problems.

Security markers comprising the use of nucleic acid molecules are also known in the art, as shown in, for example, WO 94/04918, WO 87/06383 and WO 95/02702. US 5,763,176 describes the use of multiple DNA sequences, which are attached to a bead, for marking a solid article or item. The use of multiple DNA sequences permits the introduction of marker diversity. Such security markers may be simply identified by the use of a suitable probe which may hybridise to the nucleic acid within the marker. The use of polymerase chain reaction (PCR) to isolate and multiply a sample of nucleic acid within a security marker is known, as shown in WO 90/14441. Due to PCR technology, much less of the marker needs to be recovered, therefore the spray can be done in a fine mist. An advantage of using a nucleic acid molecule as a security marker is that a unique nucleotide sequence can be used for each user of the security marker or for each location in which a security marker is used.. Use of oligonucleotides having random nucleic acid sequences is disclosed in US 5,139,812.

The inventors have realised that there is a market for improved security markers, specially designed for use in a security context. Such security markers may be used to be sprayed upon the intruder upon activation of a suitable activator. In such a case, such security markers with the appropriately designed activators and failsafes in place, can be used by the police and the Courts to prove that an intruder was at the scene of a crime at a certain time.

Where such a marker is used specifically for the purposes of crime detection it is important that the system surrounding the marker is as error-free as possible to reduce the possibility of false conviction. More specifically, the detection of the marker must be as error-free as possible, both from the possibility of cross-contamination of the marker itself to third parties and, as subject of co-pending application PCT/GB00/04419, contamination of non-marker DNA. As well as this, to be useful in the security industry to the police and the Courts, the marker needs to be designed to be flexible enough to fit with current police procedures, for example time and the holding of suspect laws.

The inventors realised that polymerase chain reaction of a strand of nucleic acid using a suitable primer, followed by sequencing from that primer, does not always produce a consistent sequence for any marker DNA. It is therefore important, in a security context, to have a backup to confirm the sequence of any unique sequence of a marker region of the security marker. The inventors have therefore provided a second primer region on the security marker which enables the sequence of the marker region to be confirmed by allowing the complementary strand to be produced by PCR and then to be sequenced using a second primer.

A further security benefit of this invention is that the busy police and forensic authorities are able to use this facility to primarily check and confirm that a marker oligo has been recovered, by matching these complimentary primers and reading the length of the unknown marker region, thus giving the police more evidence. The reduction in false positives due to contaminating DNA means that if a band of DNA having the correct size for the marker is produced, it is almost invariably due to the security marker being present and not due to contaminating DNA. This may enable the police to hold a suspect while the process of sequencing is undertaken, which in a real life forensic lab may not be immediate. This is the subject of co-pending application number PCT/GB00/04419.

One potential problem associated with security markers is that of cross-contamination to third parties, that is when the marker is passed from a person or object associated with a scene of crime to an unconnected third party. In order for the security marker to be effective in a security context, it must bind efficiently to the subject, be it a person or an object.

The inventors have designed a system that is attached to the end of the security marker which securely attaches the marker 10 the subject

One of the difficulties involved with such systems is removing the security marker to allow it to be tested and identified. The inventors have produced a security marker in which the marker region is detachable from the binding region.

Accordingly, a first aspect of the invention provides use of a security marker as set forth in Claim 1 of the present application.

In one embodiment, the security marker is a single strand of nucleic acid molecule which may be manufactured as an oligonucleotide. The nucleic acid molecule may be DNA, RNA or a synthetic nucleotide molecule.

The terms substantially "identical to a second primer" or "substantially the reverse complement of a first primer" are intended to mean that there is suffcient homology to allow the first primer or second primer, and the region to which they bind on the security marker or a nucleic acid molecule complementary to the security marker, to hybridise under high stringency conditions. Such conditions are preferably under typical PCR conditions.

Preferably, in use, the first primer is used to allow the nucleic acid molecule to replicate by PCR, thus producing a second complementary strand of nucleic acid. The complementary strand then comprises a primer region which has the correct sequence to enable the second primer to bind with it. The second primer can then be used to multiply the complementary strand of nucleic acid.

Preferably the complementary primer region is upstream of the marker region.

The inventors have also realised that where a marker has been attached to an object or an intruder, for example, there is often a large amount of contaminating DNA fragments from, for example, human beings and/or any pets, such as dogs or cat, that are around the object or intruder.

Accordingly, the nucleic acid sequence of the first primer and the nucleic acid sequence of the second primer are selected so that their respective primer binding regions do not occur with 150 bases or 120 or 100 bases of each other in native DNA. That is, naturally occurring sequences to which primers used with the security marker can bind under high stringency conditions do not normally occur within 150 bases of each other in native DNA.

By high stringency we mean, for example, 1.5 mM MgCl₂, 53°C annealing temperature.

This has been achieved by carefully selecting the primers used against publicly available databases of nucleotide sequences. Preferably the native DNA is prokaryotic or eukaryotic, especially humans, dog, cat, mouse, rat and/or insect DNA.

This has the advantage in a security context that it is possible to rapidly identify amplified DNA fragments containing the marker region by size. One or other primer may bind to non-security marker DNA but to produce a PCR product requires both primers to bind but these are likely to only produce DNA of a different size to the security marker DNA, thus enabling such fragments to be easily discounted. It also enables a forensic agency to be able to confirm quickly and easily whether they have a length of marker oligo which they can go on to sequence. This gives the police quick information enabling them to possibly hold the suspect for longer, which sequencing is undertaken, which may not take place for a few days. Plus, it may encourage the suspect to admit their guilt even before the sequencing takes place.

Once a sample containing a security marker has been identified, the security marker may then be sequenced to determine the exact sequence of the marker region. This is then matched to the database, then the premises visited to determine the time and date of the spraying.

Preferably the primers and the regions to which they bind on the security marker, or a complementary molecule, are 100% complementary to their binding region. This allows high levels of stringency to be used to assay the presence of marker.

In an especially preferred embodiment, the inventors have identified that nucleic acid from the brown throated sloth (*Bradypus variegatus*) The especially preferred sequence from the brown throated sloth used is a nucleic acid sequence corresponding to the mitochondrial ND1 gene of the sloth.

By nucleic acid we mean single or double stranded DNA or RNA of natural or synthetic origin, or synthetic derivatives thereof which may be totally or partially constituted of rare bases or by modified bases.

The primer regions are arranged on the nucleic acid to enable a suitable nucleic acid primer to selectively hybridise to the primer region, thus allowing the security marker containing the marker region to be amplified from a sample.

Preferably, the primer region consists of between 15 and 50, preferably 17-30, especially 18 nucleotides to allow sufficiently high stringency of hybridisation with a suitable primer used to identify the presence of the security marker. The primer itself may be used to directly amplify the security marker by, for example, polymerase chain reaction.

The first primer and second primer may be the same or different.

The marker region itself contains a predetermined nucleic acid sequence which is preferably unique to the owner of a particular security marker to a particular location, such as commercial premises or car. The primary use of this marker is to spray an intruder with the unique security marker.

The term "complementary" defines the relationship between two strands of DNA or DNA and RNA where they are complementary to each other if their sequences are related by base-pairing rules. Thus, ATCG is complementary to the sequence TAGC and paired with it by hydrogen bonding. Accordingly, the accuracy of the sequencing of the marker region may be determined by comparing the sequence with the sequence obtained for the marker from the second primer and seeing whether the two sequences are complementary to each other. This double checking mechanism is important for security purposes.

Preferably, the marker region consists of between 10 and 30 nucleotides, especially between 15 and 25, more preferably 20 nucleotides in length. This allows a large number of unique sequences, each of which are unique to a particular application of the security marker to be generated.

One or each primer may be separated from the marker region by a stretch of up to 50, especially between 20 and 40, more preferably a stretch of 20 to 30 nucleotides in length. The stretch of nucleotides between the primer and the marker region have been found by the inventors to improve the accuracy of the sequencing of the security marker, obviously important in this context.

It is important that the cost of the manufacturing of the oligo is not prohibitive, so reducing the market for the marker and diminishing its deterrent value. It is preferable to use shorter low volume oligo, as it is easier and cheaper to produce. Since when performing sequencing reactions, the readings tend to become clearer after about 200 mer, the sequencing results need to be further amplified for this process to be efficient. This can be achieved using a variety of methods.

When the oligo is bonded so strongly to the object, a method must be used to remove it. This may be undertaken with the use of a hairpin loop and predetermined cleavage site. Preferably, the security marker comprises at least a region of double-stranded nucleic acid. The region of double-stranded nucleic acid may be in the form of a hairpin loop. Hairpin loops are usually short double-stranded regions made possible in a single-stranded nucleic acid molecule because of the complementarity of neighbouring sequences. Preferably, the hairpin loop is formed as a result of one end of a single-stranded nucleic acid molecule having two regions of complementary nucleic acids so that a loop is formed at one end of the security marker.

Preferably, the predetermined cleavage site is a chemically labile bond, such as an acid-labile bond or a chemically reducible bond such as a disulphide bond. The cleaving of such a bond allows the separation of the portion of the security marker containing the first primer region, marker region and optionally second primer region, from the binding region. This allows the release of the portion of the nucleic acid molecule from an object to which it is bound. The released portion of the security marker may then be subjected to polymerase chain reaction and the sequence of the marker region determined.

Alternatively, the predetermined cleavage site may be a nuclease cleavage site, especially for a restriction endonuclease. Bacteria make enzymes called restriction nucleases which protect them by degrading any foreign DNA molecules. Each enzyme recognises a specific sequence of 4-6 nucleotides in DNA. Many restriction nucleases have been purified from different species of bacteria, and more than 100, most of which recognise different nucleotide sequences, are now commercially available. Examples of commonly occurring restriction endonucleases include Hpa1, Eco RI and Hind III. As indicated, such enzymes and the sites at which they cut DNA are well-known. They are readily commercially available from, for example, Promega, Southampton, United Kingdom. Protocols for using the restriction endonucleases to cut nucleic acid molecules are also very well-known and well-documented. See, for example, Promega Protocols and Applications Guide, Third Edition, 1996, pages 2-24. This reference also gives a comprehensive list of many of the known commercially available endonucleases.

Most restriction endonucleases require double-stranded DNA. Accordingly, preferably the security marker comprises at least a region of double stranded DNA which comprises a restriction endonuclease site.

The nucleic acid molecules, when exposed to the surface of any object, may be degraded by naturally occurring nucleases such as 3' or 5' exonucleases. Such nucleases may be present on the skin of a person or from bacteria on the surface on the object. It is therefore desirable to improve the resistance of the security markers to attack from such enzymes. Accordingly, preferably the nucleic acid molecule comprises at one or both ends, a blocking agent. Such blocking agents include those agents which are known to resist nuclease attack such as phosphorothioate linkage, non-natural nucleosides, dendrimers or amino alkyl groups.

Phosphorothioates are useful of binding agents because the phosphorothioate will form disulphide bonds with naturally occurring cysteine residues in proteins such as wool, hair, finger nails, etc.

The inventors have also realised that it would be possible to construct a security marker having information encoded within it on the country of origin, article to which the marker is attached or associated, etc. within the nucleic acid sequence of the marker.

Thus, in one embodiment, the marker region comprises two or more distinct coding regions to which different identifiers can be assigned.

Preferably, the security marker comprises 3, 4, 5 or more distinct coding regions.

Different coding regions allow for several different types of information to be encoded on the security marker. This is especially useful where, for example, stolen goods have been recovered by the police and they would like to identify the country of origin of the stolen goods and where they have been stolen from. Alternatively, where a solution has been sprayed on a thief during the course of a burglary or a car theft, then the coding regions can be used to identify the source of the security marker on the thief.

Accordingly, the first coding region may be assigned to identify the country of origin of the security marker. A second coding region may be assigned to identify the type of use associated with the security marker, for example as a jewellery security marker, in a car, for example a specific make of car or type of car, in a specific type of factory, bank, commercial establishment or domestic establishment, etc. A third coding region may be used to encode a specific nucleotide sequence unique to a specific firm, person or use.

When a security marker is found, the use of separate coding regions allows the source of the security marker to be rapidly identified after PCR and sequencing of the marker by looking up on a suitable records database.

The nucleic acid molecule within the security marker of the invention may be labelled. It may, for example, be labelled with a visible dye which enables the security marker to be easily seen with the naked eye. Alternatively, the label may be a fluorescent dye which enables the nucleic acid molecule to be identified under ultraviolet light.

Alternatively, the security marker may comprise one or more separate labels such as colorimetric and/or fluorescent labels.

The nucleic acid molecules, when exposed to the surface of any object, may be degraded by naturally occurring nucleases, such as 3'- or 5'-exonucleases. Such nucleases may be present on the skin of a person or from bacteria on the surface of an object. It is therefore desirable to improve the resistance of the security markers to attack from such enzymes. Accordingly, preferably the nucleic acid molecule comprises at one or both ends a blocking agent. Such blocking agents include those agents which are known to resist nuclease attack such as phosphothioate linkage, non-natural nucleosides, dendrimers or amino alkyl groups.

It is also desirable to make the security marker stick to an object onto which it has been attached. The nucleic acid molecule preferably comprises one or more binding agents which assists in attaching the nucleic acid molecule to an object. The binding agents may include biotin, vitamin-E, cholesterol and phosphothioates. Biotin, vitamin-E and cholesterol may be used since they are fat-soluble molecules which enable the nucleic acid molecule to bind to the skin of an intruder. Furthermore, biotin is especially useful because nucleic acid molecules containing biotin which have been obtained from a sample, may be readily isolated by running a solution made from the sample through an avidin-containing column. The biotin-containing nucleic acid molecule may bind to the avidin on the column, allowing it to be isolated and purified.

Phosphothioates are useful as binding-agents because the phosphothioate will form disulphide bonds with naturally occurring cysteine residues in proteins such as wool, hair, fingernails, etc.

In use, the security marker is preferably realesed by cleavage of a predetermined cleavage site. Alternatively, the security marker may be removed from the surface of an object by any suitable means. For example, swabbing using ethanol has been found to work. Skin scrapes may also be used. Dithiothreitol (DTT) has been found to be useful in isolating security markers comprising phosphothioate incorporated into the nucleic acid molecule. The DTT denatures any disulphide bonds between the phosphothioate and proteins on the object which has been marked, thus releasing the security marker.

Treating a sample with a proteinase, such as Proteinase K, has been found to be especially useful in releasing the security markers. Proteinase K, produced by the fungus *Tritirachium album* exhibits very broad cleavage specificity and digests protein in the sample. This releases the security marker and makes it available for PCR. It also digests any DNases which might be in the sample, thus preventing degradation of the marker.

Different coloured or different fluorescently labelled security markers may be used for different situations. For example, a specific colour may be used in one country and not another, thus allowing the identification of crime suspects when they pass through, for example, customs. Alternatively, different colours may be used for different situations, for example to demonstrate whether a person has carried out a car theft or has been involved in breaking and entering a factory premises.

The dye may be covalently linked to the nucleic acid molecule or may be separate.

In a preferred embodiment, the dye is made up of two colours, one having a shorter life or lever stability than the other - for example, red and blue dyes together give a purple appearance. The red dye may last on the skin for several days and the blue for several weeks. If the suspect has the purple dye, therefore, the police know that the spray has been dispersed in the last few days. A blue dye will indicate the spray was activated a longer time ago. Different colours may also indicate a different type of alarm system. For example, commercial properties may have purple dye, vehicles orange dye (red and yellow). The police can then check their records (as they are waiting for the results of the forensic tests) for the appropriate time period and type of crime committed. Preferably the dyes are only visible under UV light.

Alternatively, IR dyes can be used. They are invisible in natural light, but glow green in IR. They are particularly useful if used in conjunction with IR LEDs, for example in CCTV systems, to follow a suspect bearing the dye.

A method of marking an object comprising applying a security marker to an object is also provided. A security marker may be applied by any suitable means such as using a brush, a spray, an aerosol or a stream. Preferably, the object is a human, such as an intruder.

According to another aspect of the invention, there is provided a method of detecting a security marker as set forth in claim 18.

The security marker may be amplified by, for example, PCR prior to sequencing the first marker region.

The security marker may additionally be screened with a second probe capable of hybridising to the second primer region once the marker has been replicated to form a complementary strand by e.g. PCR, and the second marker region is then sequenced.

The marker regions may be sequenced by any methods known in the art, such as dideoxy sequencing, mass-spectrometry sequencing or other techniques known in the art.

Such sequencing may occur after initial amplification by PCR or directly, e.g. using minisequencing.

The sequence of marker region may be compared with a database to determine the source of the security marker. That is, to enable the owner of the object to be identified, for example, or to identify where a prime suspect may have been exposed to the security marker.

A security marker according to the first aspect of the invention may be included as part of a security kit. The security kit may comprise a security marker within a suitable solution or, for example, within a grease. The grease may be of use to ensure that a sample of cloth is successfully marked.

The kit may comprise aerosol means for applying the security marker as a spray or aerosol onto an object. The security system may additionally comprise a smoke generator. Such smoke generators use, for example, a glycol to rapidly fill a space, such as the inside of a room or a car, with a chemical smoke. This disorientates an intruder and prevents the intruder making off with objects or the car. The security system may also comprise suitable sensors and actuators to actuate the application of the security marker onto an intruder. The actuator may also be activated remotely, for example from a control room.

The inventors have also realised that it is possible to improve the coating of an object or person subjected to a spray or aerosol of a security marker according to the invention. They found that it is possible to do this by applying an electrostatic charge to the aerosol or smoke. Accordingly, a further aspect of the invention provides an applicator for a security marker as set forth in claim 15.

The marker may be in solution, a dispersion or in a powder.

The means for generating an electrostatic charge may be a charged plate over which the security marker is forced prior to exiting the nozzle of the applicator. The charge plate may have a negative or positive charge in order to add or remove electrons from the spray or aerosol.

Charging the spray or aerosol makes the spray or aerosol stick to objects, thus improving coverage with the security marker.

Details of where a security marker, according to the invention, is in use and a list of the nucleic acid sequence encoding the marker region of the security marker may be included as part of a database. This enables the source of a security marker, according to the invention, to be identified.

An actuator may be employed as part of a security device for releasing a supply of nucleic acid according to the invention and additionally comprising a write once read only memory (ROM) to record the date and time of an activation of the actuator. Such a device enables the exact date and time of the entry of an intruder to be determined. It is useful in a security context as the recording cannot be changed after the event. This improves the probability that the intruder will be successfully prosecuted by providing an exact record of the circumstances surrounding the covering of the intruder with the security marker.

A camera and image recording device, known as a "snapshot", may also be employed to take one or more images of the release of the security marker according to the invention onto a person or item.

This will work alongside the DNA activation, so not only marking the person, but also taking a picture of the person as they are marked. The snapshot board has no moving parts and so is extremely reliable. It works by recording picture images on a cyclic loop continuously. These cannot be accessed until an event occurs - such as the DNA being sprayed. Then a number of images before the spraying, the spraying itself and a number of images after the spraying are stored, stamped with the time, date and the identity number of the linked DNA solution as shown on the database. Only the appropriate authorities - i.e. the police, can then retrieve the images. The authorities can use this tool in two ways. The snapshot can help the police by letting them know the suspect's appearance. This can be stored on a database. When a suspect is apprehended with, e.g. the UV dye on them, before the DNA is even read, they may go to the database and retrieve a picture of the suspect in the premises, so securing a conviction. Plus, if the image is obscured in any way, there will also be the DNA evidence from the scene, once it has been sequenced.

The invention will now be described by way of example only with reference to the following figures.
**Figure 1** shows a schematic diagram representing a security marker according to the first aspect of the invention.
**Figure 2** shows examples of restriction endonuclease sites for Hpa I;
   Eco RI; and
   Hind III.
**Figure 3** shows a schematic diagram representing a security marker after it has been separated from the binding region.

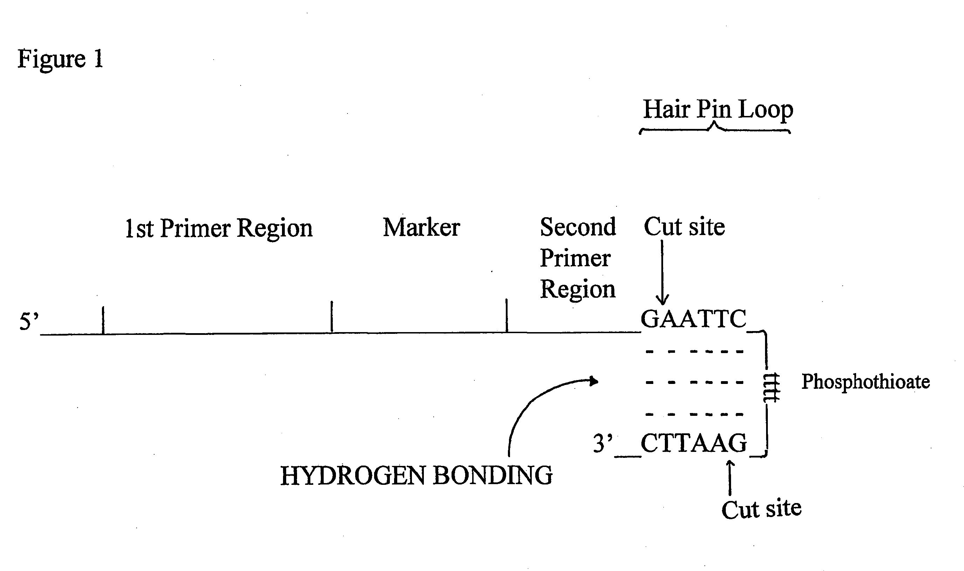

Referring to Figure 1, this shows a security marker according to the first aspect of the invention. It comprises a single-stranded piece of nucleic acid which comprises a hairpin loop region. The hairpin loop is formed because the single-stranded nucleic acid molecule comprises two sequences of nucleic acids which are complementary to one another. This allows the single-stranded nucleic acid molecule to fold around on itself to form a hairpin loop. The hairpin loop is maintained in its structure by hydrogen bonding between complementary nucleic acids (G-C and A-T). The hairpin loop structure additionally contains several phosphothioate bonds (shown as tttt). Phosphothioate has been found to bind to the skin of people and also to fibres, such as wool. This enables the security marker to be applied to objects and, for example, to thieves or other people who have triggered a security device containing the security marker. The "palindromic" sequence of nucleic acid (gaattc-cttaag) encodes for an Eco RI restriction endonuclease site. This means that where a sample of fabric, or for example a skin swab, has been recovered from a scene of crime, Eco RI can be used to separate the region of the nucleic acid encoding the first primer region and the second primer region, together with the marker, for use in polymerase chain reaction to multiply the sample to a size where it can be sequenced. It should be emphasised that the example shown in Figure 1 is not limiting, and other restriction endonuclease sites may be used.

An example of a construct according to Figure 1 is shown below: 
5'_GAATTCGAGCATAATACCCACGATTCCGCTACGATCAACTA/ATACAC
TTGGTATGAAACAATTT/CCTCCCACTCACCCTGGCCCTATGCACGTGACA
CATAACCGAATTCTTttttTTGAATTC_3'
/ \
double stranded looped area
 where: tttt is a phosphothioate bond.

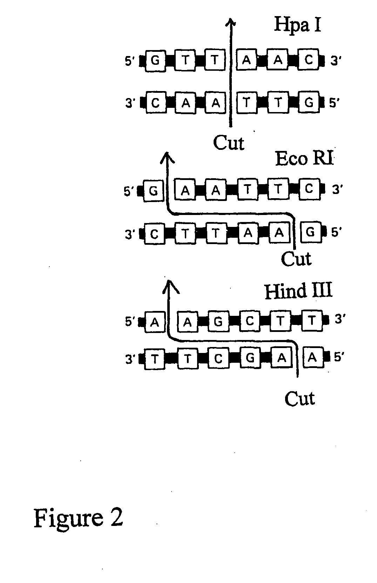

This has been successfully removed by the inventors using standard restriction endonuclease protocols from skin and fabric. The restriction endonuclease protocols are shown is, for example, the Protocols and Applications Guide from Promega (Supra). Restriction endonuclease sites are also shown, for example, in Figure 2.

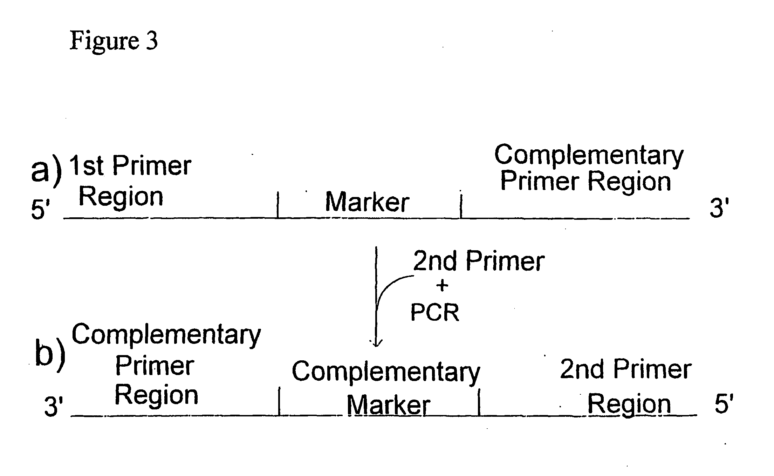

Figure 3 shows a schematic diagram showing the use of a detached region of nucleic acid molecule to sequence and identify a specific marker sequence. A typical protocol for the identification of the marker sequence is given below:

Figure 3a shows a single-stranded nucleic acid molecule. The nucleic acid molecule may be of DNA or RNA. The strand comprises a first primer region and, downstream of the primer region, a marker region and a complementary primer region. Upon carrying out PCR with a primer capable of binding the second primer region, a complementary strand (1b) is produced. The complementary strand has a functional first primer region capable of being bound by a first primer. It also has a marker region complementary to the marker region on the original marker.

The inventors have found that a particularly useful primer sequence may be obtained from the brown-throated sloth. This has the following sequence:

5'-ta cccacgattccgctacgatc aactaataca cttgctatga aaaaatttcc tcccactcac
cctggccctatgca cgtgacacataacc-3'

This is obtained from the mitochondrial ND1 gene of the organism.

This was used to create the following primers:
1. 5'-ta cccacgattccgctac-3'.
2. 5'-ggttatgtgtcacgtgca-3'.

The primer sequences were attached to unique marker sequences, for example of sequence:
gaaaaaatttcctcccactcac
which were separated from the primer sequences by a stretch of 22 bases from one primer region, 10 bases from a second primer region.

The nucleic acid molecules can be prepared by conventional solid-phase methods, typically on an automated DNA synthesiser. The most common method involves the use of β-cyanoethyl phosphoramidite chemistry as described in the following publications:
The synthesis of chemically labelled PCR primers. D.J.S. Brown and T. Brown (1995 "PCR: essential data" Wiley, ed. C.R. Newton. Chapter 7, pp 57-70.
Preparation of synthetic oligodeoxynucleotide probes. T. Brown and J. Grzybowski (1995) "Gene probes: a Practical Approach". IRL press. chapter 5, 146-167.

Oligonucleotides can also be synthesised by manual solid-phase methods or by solution-phase methods but the automated solid-phase method has the advantage of high throughput.

DNA isolated from biological sources can also be used.

### Polymerase Chain Reaction Protocol

Conventional PCR techniques were used to identify and multiply the sequences. Typical 50 µL reaction mix comprise.
10x PCR buffer developed for SYBR Green reactions on the ABI Prism Sequence detection system 7700, which was obtained from Genesys.
200 µM dNTP's (using a mixture of dUTP and dTTP)
0.5 µM concentration of both primers.
1.5 mM MgCl₂.
1 unit of Hot GoldStar enzyme (a TAQ polymerase)
0.5 units of UNG.

Typical cycling conditions were 53°C for 2 mins., 95°C for 10 mins., 40 cycles of 95° for 30 secs., 50° for 30 secs. and then 72°C for 30 secs. A final extension was carried out at 72°C for 7 mins.

Marker DNA can then be sequenced using conventional sequencing techniques.

### Proteinase K Release Protocol

Security markers of the invention have been successfully released from samples of skin, fibre and hair using Proteinase K. The enzyme is commercially available from, e.g. Promega UK, Southampton, UK. Typically, Proteinase K is added to 0.2 mg./ml. incubated at 56°C overnight, and then ethanol precipitated to isolate the security marker.

### Dyes

Appropriate dyes may be organic or inorganic dyes.

## Claims

1. Use of a security marker for marking objects and people, wherein said security marker comprises:
(i) a first primer region substantially the reverse complement of a first primer and wherein said first primer region is capable of being bound by the first primer;
(ii) linked to the first primer region, a marker region comprising a predetermined nucleic acid sequence capable of identifying the source of the security marker: and
(iii) a second primer region substantially identical to a second primer, wherein the second primer region is linked to the marker region;
wherein the nucleic acid sequence of the first primer region and the nucleic acid sequence of the second primer region do not occur within 100 bases of each other in a native DNA sequence from humans, dog, cat, mouse, rat, insects or prokaryotic organisms;
(iv) a binding region for attaching the marker to an object or person; and
(v) a predetermined cleavage site for allowing the separation of the first primer region, the marker region and the second primer region from the binding region.

2. Use according to Claim 1, comprising at least a region of double-stranded nucleic acid.

3. Use according to Claim 2, wherein the region of double-stranded nucleic acid is in the form of a hairpin loop.

4. Use according to Claim 3, wherein the binding region is at the loop end of the hairpin loop.

5. Use according to any preceding claim wherein the predetermined cleavage site is a chemically labile bond or a restriction endonuclease cleavage site.

6. Use according to Claim 5, wherein the predetermined cleavage site is cleavable by Ecor RI restriction endonuclease and comprises a double-stranded nucleic acid sequence:
- G'AATTC-
- CTTA'G-
where " ' " indicates the cleavage site.

7. Use according to any preceding claim wherein the binding region comprises biotin, vitamin E, cholesterol or phosphothioate.

8. Use according to any preceding claim, additionally comprising a dye.

9. Use according to Claim 8, wherein comprising a dye which is visible under ultraviolet or infrared light.

10. Use according to Claim 8 or Claim 9 comprising two dyes which degrade at different rates in the environment.

11. Use according to any preceding claim comprising linked to the first primer region a marker region comprising two or more distinct coding regions to which different identifiers can be assigned.

12. Use according to Claim 11 additionally comprising, linked to the marker region, a second primer region substantially identical to a second primer.

13. Use according to Claim 11 or Claim 12 comprising 3 distinct coding regions.

14. Use according to Claim 13, wherein a first coding region is assigned to identifying the country of origin of an article to which the marker is associated; a second coding region is assigned to identifying the type of article to which the marker is associated, and the third region is assigned to a unique nucleic acid code associated with the specific marker.

15. An applicator for a security marker comprising:
(i) a reservoir containing a security marker as defined in any preceding claim;
(ii) pressurisation means for forcing the security marker through a nozzle to form a spray or aerosol, **characterised in that**;
(iii) the applicator comprises means to generate an electrostatic charge on the spray or aerosol of the security marker.

16. Use according to Claim 1, wherein the first primer region, the marker region and the second primer region comprise the sequence 5'-ta cccacgattccgctacgatc aactaataca cttgctatga aaaaatttcc tcccactcac cctggccctatgca cgtgacacataacc-3'.

17. Use according to Claim 1, wherein the security marker comprises the sequence 
5'-GAATTCGAGCATAATACCCACGATTCCGCTACGATCAACTAATACACTT
GGTATGAAACAATTTCCTCCCACTCACCCTGGCCCTATGCACGTGACACA
TAACCGAATTCTTttttTTGAATTC-3',
 wherein tttt is a phosphothioate bond.

18. A method of detecting a security marker as defined in any of claims 1-14, 16 or 17 when said security marker is bound via the binding region to an object or a person, said method comprising:
(i) cleaving the predetermined cleavage site and releasing a portion of the security marker nucleic acid comprising the first primer region, the marker region and the second primer region;
(ii) amplifying the portion of the security marker nucleic acid sequence with first and second primers, wherein in use the first primer binds to the first primer region and the second primer binds to a sequence complementary to the second primer region; and
(iii) identifying amplified nucleic acid fragments containing the portion of the security marker.

19. A security marker comprising the nucleic acid sequence 
5'-GAATTCGAGCATAATACCCACGATTCCGCTACGATCAACTAATACACT
TGGTATGAAACAATTTCCTCCCACTCACCCTGGCCCTATGCACGTGACAC
ATAACCGAATTCTTttttTTGAATTC-3',
 wherein tttt is a phosphothioate bond.

## Patentansprüche

1. Verwendung eines Sicherheits-Markers zur Markierung von Gegenständen und Personen, wobei der Sicherheits-Marker folgendes umfasst:
(i) einen ersten Primer-Bereich, welcher im wesentlichen das reverse Komplement eines ersten Primers darstellt, und wobei der erste Primer-Bereich in der Lage ist, von dem ersten Primer gebunden zu werden;
(ii) verknüpft mit dem ersten Primer-Bereich einen Marker-Bereich, der eine zuvor festgelegte Nukleinsäure-Sequenz umfasst, welche in der Lage ist, die Quelle des Sicherheits-Markers zu identifizieren; und
(iii) einen zweiten Primer-Bereich, der im wesentlichen identisch ist mit einem zweiten Primer, wobei der zweite Primer-Bereich mit dem Marker-Bereich verknüpft ist;
wobei die Nukleinsäure-Sequenz des ersten Primer-Bereichs und die Nukleinsäure-Sequenz des zweiten Primer-Bereichs in einer nativen DNA-Sequenz, ausgewählt aus Mensch, Hund, Katze, Maus, Ratte, Insekten oder prokaryotischen Organismen, nicht innerhalb eines Abstandes von 100 Basen voneinander auftreten;
(iv) ein Bindungsbereich zur Befestigung des Markers an einen Gegenstand oder eine Person; und
(v) eine zuvor festgelegte Schnittstelle, um die Abtrennung des ersten Primer-Bereichs, des Marker-Bereichs und des zweiten Primer-Bereichs vom Bindungsbereich zu ermöglichen.

2. Verwendung gemäß Anspruch 1, umfassend mindestens einen Bereich mit einer doppelsträngigen Nukleinsäure.

3. Verwendung gemäß Anspruch 2, wobei der Bereich der doppelsträngigen Nukleinsäure in der Form einer Haarnadelschleife (hairpin loop) vorliegt.

4. Verwendung gemäß Anspruch 3, wobei der Bindungsbereich am Ende der Schleife der Haarnadelschleife liegt.

5. Verwendung gemäß einem der vorhergehenden Ansprüche, wobei die zuvor festgelegte Schnittstelle eine chemisch labile Bindung oder eine Restriktionsendonuklease-Schnittstelle ist.

6. Verwendung gemäß Anspruch 5, wobei die zuvor festgelegte Schnittstelle durch die Restriktions-Endonuklease-Ecor RI spaltbar ist und eine doppelsträngige Nukleinsäure-Sequenz umfasst:
- G'AATTC-
- CTTA'G-
wobei " ' " die Schnittstelle angibt.

7. Verwendung gemäß einem der vorhergehenden Ansprüche, wobei der Bindungsbereich Biotin, Vitamin E, Cholesterin oder Phosphorthioat umfasst.

8. Verwendung gemäß einem der vorhergehenden Ansprüche, zusätzlich umfassend einen Farbstoff.

9. Verwendung gemäß Anspruch 8, umfassend einen Farbstoff, der unter ultraviolettem oder infrarotem Licht sichtbar ist.

10. Verwendung gemäß Anspruch 8 oder Anspruch 9, umfassend zwei Farbstoffe, welche unterschiedlich schnell in der Umwelt abgebaut werden.

11. Verwendung gemäß einem der vorhergehenden Ansprüche, umfassend einen Marker-Bereich, der mit dem ersten Primer-Bereich verknüpft ist, umfassend zwei oder mehr verschiedene kodierende Bereiche, denen unterschiedliche Kennungen zugeordnet werden können.

12. Verwendung gemäß Anspruch 11, zusätzlich umfassend einen zweiten Primer-Bereich, der mit dem ersten Marker-Bereich verknüpft ist, und der im wesentlichen mit einem zweiten Primer identisch ist.

13. Verwendung gemäß Anspruch 11 oder Anspruch 12, umfassend 3 verschiedene kodierende Bereiche.

14. Verwendung gemäß Anspruch 13, wobei ein erster kodierender Bereich der Identifizierung des Ursprungslandes eines Artikels, mit dem der Marker verknüpft ist, zugeordnet wird; ein zweiter kodierender Bereich der Identifizierung des Artikeltyps, mit welchem der Marker verknüpft ist, zugeordnet wird, und der dritte Bereich einem einzigartigen Nukleinsäure-Code zugeordnet wird, der mit dem spezifischen Marker verknüpft ist.

15. Eine Auftragungsvorrichtung für einen Sicherheits-Marker, umfassend:
(i) ein Reservoir, enthaltend einen Sicherheits-Marker wie in einem der vorhergehenden Ansprüche definiert;
(ii) ein Mittel zur Erzeugung von Druck, um den Sicherheits-Marker durch eine Düse zur Bildung eines Sprays oder Aerosols zu zwingen, **dadurch gekennzeichnet, dass**;
(iii) die Auftragungsvorrichtung Mittel zur Erzeugung einer elektrostatischen Ladung beim Spray oder Aerosol des Sicherheits-Markers umfasst.

16. Verwendung gemäß Anspruch 1, wobei der erste Primer-Bereich, der Marker-Bereich und der zweite Primer-Bereich die Sequenz 5'-tacccacgattccgctacgatc aactaataca cttgctatga aaaaatttcc tcccactcac cctggccctatgca cgtgacacataacc-3' umfasst.

17. Verwendung gemäß Anspruch 1, wobei der Sicherheits-Marker die Sequenz 
5'-GAATTCGAGCATAATACCCACGATTCCGCTACGATCAACTAATACACTTGG
TATGAAACAATTTCCTCCCACTCACCCTGGCCCTATGCACGTGACACATAACC
GAATTCTTttttTTGAATTC-3' umfasst,
 wobei tttt eine Phosphorthioat-Bindung darstellt.

18. Verfahren zum Detektieren eines Sicherheits-Markers wie in einem der Ansprüche 1-14, 16 oder 17 definiert, wenn der Sicherheits-Marker über den Bindungsbereich an einen Gegenstand oder eine Person gebunden wird, wobei das Verfahren folgende Schritte umfasst:
(i) Spaltung der zuvor festgelegten Schnittstelle und Freisetzen eines Anteils der Nukleinsäure des Sicherheits-Markers, umfassend den ersten Primer-Bereich, den Marker-Bereich und den zweiten Primer-Bereich;
(ii) Amplifizieren des Anteils der Nukleinsäure-Sequenz des Sicherheits-Markers mit ersten und zweiten Primern, wobei bei der Verwendung der erste Primer an den ersten Primer-Bereich bindet und der zweite Primer an eine Sequenz bindet, die zum zweiten Primer-Bereich komplementär ist; und
(iii) Identifizierung der amplifizierten Nukleinsäure-Fragmente, die den Anteil des Sicherheits-Markers enthalten.

19. Sicherheits-Marker, umfassend die Nukleinsäure-Sequenz 
5'-GAATTCGAGCATAATACCCACGATTCCGCTACGATCAACTAATACACTTGG
TATGAAACAATTTCCTCCCACTCACCCTGGCCCTATGCACGTGACACATAACC
GAATTCTTttttTTGAATTC-3',
 wobei tttt eine Phosphorthioat-Bindung darstellt.

## Revendications

1. Utilisation d'un marqueur de sécurité pour marquer les objets et les personnes, ledit marqueur de sécurité comprenant :
(i) une première région amorce qui est sensiblement le complément inverse d'une première amorce et ladite première région amorce étant capable d'être liée par la première amorce ;
(ii) une région marqueur, liée à la première région amorce, comprenant une séquence d'acides nucléiques prédéterminée capable d'identifier la source du marqueur de sécurité ; et
(iii) une seconde région amorce sensiblement identique à une seconde amorce, la seconde région amorce étant liée à la région marqueur ;
dans laquelle la séquence d'acides nucléiques de la première région amorce et la séquence d'acides nucléiques de la seconde région amorce n'apparaissent pas à moins de 100 bases l'une de l'autre dans une séquence d'ADN native provenant d'humains, de chiens, de chats, de souris, de rats, d'insectes ou d'organismes procaryotes ;
(iv) une région de liaison pour attacher le marqueur à un objet ou une personne ;
(v) un site de clivage prédéterminé pour permettre la séparation de la première région amorce, la région marqueur et la seconde région amorce de la région de liaison.

2. Utilisation selon la revendication 1, comprenant au moins une région d'acides nucléiques double brin.

3. Utilisation selon la revendication 2, dans laquelle la région d'acides nucléiques double brin a la forme d'une boucle en épingle à cheveux.

4. Utilisation selon la revendication 3, dans laquelle la région de liaison est à l'extrémité de boucle de la boucle en épingle à cheveux.

5. Utilisation selon l'une quelconque des revendications précédentes, dans laquelle le site de clivage prédéterminé est une liaison chimiquement labile ou un site de clivage d'une endonucléase de restriction.

6. Utilisation selon la revendication 5, dans laquelle le site de clivage prédéterminé peut être clivé par l'endonucléase Ecor RI et comprend une séquence d'acides nucléiques double brin :
- G'AATTC-
- CTTA'G-
où " ' " indique le site de clivage.

7. Utilisation selon l'une quelconque des revendications précédentes, dans laquelle la région de liaison comprend la biotine, la vitamine E, le cholestérol ou le phosphothioate.

8. Utilisation selon l'une quelconque des revendications précédentes, comprenant de plus un colorant.

9. Utilisation selon la revendication 8, comprenant un colorant qui est visible sous une lumière ultraviolette ou à infrarouge.

10. Utilisation selon la revendication 8 ou la revendication 9, comprenant deux colorants qui se dégradent à des vitesses différentes dans l'environnement.

11. Utilisation selon l'une quelconque des revendications précédentes comprenant, liée à la première région amorce, une région marqueur comprenant deux régions codantes distinctes ou plus auxquelles on peut attribuer deux identifiants différents.

12. Utilisation selon la revendication 11, comprenant de plus, liée à la région marqueur, une seconde région amorce sensiblement identique à une seconde amorce.

13. Utilisation selon la revendication 11 ou la revendication 12, comprenant trois régions codantes distinctes.

14. Utilisation selon la revendication 13, dans laquelle une première région codante est attribuée pour identifier l'origine d'un article auquel le marqueur est associé ; une seconde région codante est attribuée pour identifier le type d'article auquel le marqueur est associé, et la troisième région est attribuée à un code d'acides nucléiques unique associé au marqueur spécifique.

15. Applicateur pour un marqueur de sécurité comprenant :
(i) un réservoir contenant un marqueur de sécurité tel que défini dans l'une quelconque des revendications précédentes ;
(ii) un moyen de pressurisation pour forcer le marqueur de sécurité au travers d'une buse pour former un pulvérisateur ou un aérosol, **caractérisé en ce que** ;
(iii) l'applicateur comprend un moyen pour générer une charge électrostatique sur le pulvérisateur ou l'aérosol du marqueur de sécurité.

16. Utilisation selon la revendication 1, dans laquelle la première région amorce, la région marqueur et la seconde région amorce comprennent la séquence 5'tacccacgattccgctacgatc aactaataca cttgctatga aaaaatttcc tcccactcac cctggccctatgca cgtgacacataacc-3'.

17. Utilisation selon la revendication 1, dans laquelle le marqueur de sécurité comprend la séquence 
5'-GAATTCGAGCATAATACCCACGATTCCGCTACGATCAACTAATACACTT
GGTATGAAACAATTTCCTCCCACTCACCCTGGCCCTATGCACGTGACACA
TAACCGAATTCTTttttTTGAATTC-3',
 dans laquelle tttt est une liaison phosphothioate.

18. Procédé de détection d'un marqueur de sécurité tel que défini dans l'une quelconque des revendications 1 à 14, 16 ou 17, quand ledit marqueur de sécurité est lié par la région de liaison à un objet ou une personne, ledit procédé comprenant :
(i) le clivage du cycle de clivage prédéterminé et la libération d'une partie de l'acide nucléique du marqueur de sécurité comprenant la première région amorce, la région marqueur et la seconde région amorce ;
(ii) l'amplification de la partie de la séquence d'acides nucléiques du marqueur de sécurité avec les première et seconde amorces, dans lequel, lors de l'utilisation, la première amorce se lie à la première région amorce et la seconde amorce se lie à une séquence complémentaire à la seconde région amorce ; et
(iii) l'identification de fragments d'acides nucléiques amplifiés contenant la partie du marqueur de sécurité.

19. Un marqueur de sécurité comprenant la séquence d'acides nucléiques 
5'-GAATTCGAGCATAATACCCACGATTCCGCTACGATCAACTAATACACT
TGGTATGAAACAATTTCCTCCCACTCACCCTGGCCCTATGCACGTGACAC
ATAACCGAATTCTTttttTTGAATTC-3',
 dans laquelle tttt est une liaison phosphothioate.
